(19) Europäisches Patentamt
European Patent Office
Office européen des brevets

(11) **EP 2 545 881 B1**

(12) **EUROPEAN PATENT SPECIFICATION**

(45) Date of publication and mention
of the grant of the patent:
**21.06.2017 Bulletin 2017/25**

(51) Int Cl.:
**A61C 8/00** *(2006.01)* **A61L 27/10** *(2006.01)*
**C04B 35/486** *(2006.01)* **C04B 35/488** *(2006.01)*

(21) Application number: **12175922.9**

(22) Date of filing: **11.07.2012**

(54) **Implant**

Implantat

Implant

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.07.2011 JP 2011157000
05.03.2012 JP 2012048124**

(43) Date of publication of application:
**16.01.2013 Bulletin 2013/03**

(73) Proprietor: **SHOFU INC.**
**Kyoto-shi, Kyoto 605-0983 (JP)**

(72) Inventors:
• **Kashiwabara, Tateki**
 **Kakamigahara-shi, Gifu 509-0103, (JP)**
• **Goto, Tetsuro**
 **Kakamigahara-shi, Gifu 509-0103, (JP)**
• **Deguchi, Mikito**
 **Kyoto-shi, Kyoto 605-0983, (JP)**
• **Yoshimoto, Ryuichi**
 **Kyoto-shi, Kyoto 605-0983, (JP)**
• **Hori, Koji**
 **Kyoto-shi, Kyoto 605-0983, (JP)**
• **Ito, Michio**
 **Shiojiri, Nagano 399-6462 (JP)**

(74) Representative: **Addiss, John William et al**
 **Mewburn Ellis LLP**
 **City Tower**
 **40 Basinghall Street**
 **London EC2V 5DE (GB)**

(56) References cited:
**EP-A1- 2 263 991** **EP-A1- 2 316 374**
**WO-A1-2011/064369** **JP-A- 2002 362 972**
**US-A1- 2009 321 971**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to an implant fixture typically used in the field of dentistry, especially in the field of artificial tooth roots.

In the recent years, public attention has been paid to implant technology by which an implant fixutre such as an artificial tooth root is implanted in a living organism, thereby restoring a lost function.

In the field of dentistry, for example, a fossa for implantation of an artificial tooth root is formed with a drill or the like in a predetermined size in a jawbone after cutting open the gingiva of a tooth lost portion. An implant fixture is placed into the fossa. Then, a certain period of time is allowed for the surface of the implant fixture to integrate or fuse with the contacting surface of the jawbone at a micro level. This is called osseointegration. Follwoing that, a superstructure or an upper structure (a crown) is mounted on the implant fixture directly or via an abutment.

In circumstances, spcifically in the mouth, where a dental implant fixture is used, dental caries bacteria adhere to the tooth surface together with plaque and produce an organic acid such as lactic acid from carbohydrate or sugar, thereby decalcifying the tooth structure. The dental implant fixture is used in special circumstances where the implant fixture is exposed to an acid enough to cause decalcification of the tooth structure, compared with other prostheses such as artifical bones and joints. Thus, the dental implant fixture is required to have especially high durability, specifically high lactic acid resistance. In addition to the high durability, high performance in osseointegration, strength, and safety is called for.

Dental implant fixtures made from ceramics mainly composed of zirconia have been attracting public attention in the recent years (refer to JP 2002-362972 A). The ceramic implant fixtures are excellent in strength. Further, compared to metallic implant fixtures, ceramic implant fixtures are excellent in safety since they do not cause allergic reactions to metal.

Conventional ceramic implant fixures have hardly attained both high duralibity and good osseointegration. Conventionally, surface finising or surface treatment to provide appropriate surface roughness is required to improve osseointegration. For example, a titanium implant fixture needs surface finishing by sandblasting, acid treatment or both. If a ceramic implant fixture is subjected to such surface finishing, monoclinic crytalline structure is exposed on the surface of the implant fixture, thereby reducing the durability of the implant fixture.

If the ceramic implant fixture is not subjected to such surface finishing and the surface roughness is accordingly inappropriate, the degree of osseointegration is decreased.

Document EP 2 316 374 A1 discloses an implant fixture made from zirconia. In the process of making the ceramic body roughness, particles of a ceramic material are deposed on the surface of a ceramic basic body to avoid creation of monoclinic phase as compare to sandblasting.

In view of the above-mentioned technical problems, the present invention has been made. Accordingly, a preferred aim of the present invention is to provide an implant fixture having high durability and capable of excellent osseointegration.

An implant fixture of the present invention, as disclosed in claim 1, is made from ceramics containing zirconia, and has monoclinic percentage or percentage of monoclinic crystals of 1 volume % or less. The implant fixture comprises a buried portion having an arithmetic average roughness Ra of 1 to 5 $\mu$m.

The implant fixture of the present invention is excellent in resistance against lactic acid or the like since the monoclinic crystals or monoclinic crystalline structure accounts for 1 volume % or less, preferably 0.5 volume % or less, and more preferably 0 volume % of the total volume of the fixture.

The buried portion of the implant fixture has an arithmetic average roughness Ra in the range of 1 to 5 $\mu$m. This assures robust osseointegration between the bone and the fixture. Preferably, the maximum height Rz of the profile of the implant fixture is in the range of 5 to 40 $\mu$m.

Further, the implant fixture of the present invention has high affinity and remarkable compatibility with a living body (high bioaffinity and remarkable biocompatibility). Based on clinical testing, the implant fixture of the present invention evidently shows a significant difference with other implant fixtures.

According to the present invention, the zirconia content accounts for 86 mass % or more, preferably 89 mass % or more, and more preferably 92 mass % or more of the total mass of the implant fixture. If the zirconia content falls within this range, the resistance against lactic acid or the like may further be increased.

Preferably, the implant fixture contains alumina. As a result, dense ceramics may be obtained even with a low burning temperature. If alumina is not contained in the ceramics, dense ceramics may be obtained with a high burning temperature, but the sintered grain size of the ceramics becomes large. If the burning temperature is lowered, the sintered grain size becomes small, but ceramic density decreases. The alumina content is preferably in the range of 0.05 to 3 mass %, more preferably 0.05 to 1 mass %, and further preferably 0.05 to 0.1 mass % of the total mass of the implant fixture.

The implant fixture of the present invention preferably contains at least one sort selected from the group of yttria, ceria, magnesia, and calcia. Especially, it is preferable that the implant fixture contains yttria. Inclusion of one or more of these components may stabilize the contained zirconia in a tetragonal state. This, in turn, may suppress the surface of the implant fixture from crystallizing in the monoclinic system, thereby readily obtaining an implant fixture with low monoclinic percentage. This may also suppress crystallizing in the monoclinic system under the circumstances where the implant fixture is exposed to lactic acid and hot water, thereby increasing the durability of the implant fixture. If yttria is contained, its content is preferably in the range of 2 to 4 mol %.

The implant fixture preferably has a sintered grain size of 0.45 $\mu$m or less, more preferably 0.3 $\mu$m or less, and further preferably 0.009 to 0.3 $\mu$m. In this range of the sintered grain size, the resistance against lactic acid or the like may furthermore be increased. The sintered grain size is measured by planimetric method.

The implant fixture may contain minor components other than zirconia, alumina, yttria, ceria, magnesia, and calcia.

The ceramics forming the implant fixture are preferably dense, which may increase the resistance against lactic acid or the like and attain sufficient strength. The relative density of the ceramics is preferably 95% or more, more preferably 98% or more, and further preferably 99% or more.

Preferably, the implant fixture of the present invention has a surface that is substantially not subjected to annealing treatment. The term "annealing treatment" used herein means that sintered ceramics are subjected to heating with a high temperature of 800°C or more after being subjected to cutting, polishing, blasting or other working. The annealing treatment reduces monoclinic crystals occurring on the worked surface of the sintered ceramics, but likely worsens the durability compared to a non-worked sintered surface.

The implant fixture of the present invention is typically manufactured by the following steps. In short, a slurry of ceramics containing zirconia is poured into a mold for the implant fixture and then the ceramics are let hardened.

According to the above-mentioned method, there is no need of cutting the shape of the implant fixture out of the sintered ceramics in a lump form. The monoclinic percentage hardly increases in the implant fixture. As a result, the manufactured implant fixture may have high durability.

In this manufacturing method, the surface roughness of the implant fixture may be determined by setting the surface roughness of an inner surface of the mold that contacts the slurry to a predetermined value.

For example, the surface roughness of the inner surface of the mold may be determined by blasting the inner surface of the mold. Alternatively, the surface of a master model is subjected to blasting and then the surface roughness of the master model is transferred to the inner surface of the mold. Sandblast media used in blasting have an average grain size of 50 to 500 $\mu$m, preferably 80 to 300 $\mu$m.

The blast media may be based on alumina, silicon carbide, and zirconia. The blast media typically include steel shot, steel grit, microshot, peening shot, SB ultra-hard shot, advanced shot, bright shot, stainless shot, aluminum cut wire, AMO beads, glass beads, glass powder, Alundum, carborundum, ceramic beads, nylon shot, polycarbonate, melamine, urea, walnut shot, apricot, and peach. Selection from these media is arbitrary. Sandblasters such as general suction sandblasters, general direct pressure sandblasters, small-sized recirculating sandblasters, barrel-type small-sized recirculating sandblasters, and pen-type sandblasters are available. A pen-type sandblaster may preferably be used in detailed blasting.

A typical blast pressure is 0.2 to 1.2 Kgf/cm$^2$, depending upon the material and grain size of the blast media used.

A slurry used in the above-mentioned manufacturing method contains, for example, ceramic powder and binders for hardening the slurry. The slurry may also contain a water soluble polymer for viscosity adjustment, various solvents, and surface active agents for ready dispersion and wetting.

The binders used herein typically includes thermosetting binders such as epoxy resin, polyester, phenol resin, melamine resin, polyimide, cyanate ester resin, diallyl phthalate resin, silicone resin, isocyanate resin, and modified resins of these resins. Emulsions of these resins may alternatively be used. Further, thermal-gelation binders such as protein and starch may be used.

A solvent for the slurry is, for example, water, aromatic solvent, aliphatic solvent, ester, or ketone-based solvent. The slurry may be prepared by mixing the ceramic powder, binder and other components in the solvent, sufficiently dispersing and kneading them using a ball mill, and then performing vacuum defoaming.

The mold used in the above-mentioned manufacturing method is preferably made of elastically deformable and stretchable material. Thus, the mold may be deformed accdording to the shape, even a complex shape, of the implant fixture, thereby enabling the implant fixture to be readily taken out of the mold. The material of the mold typically includes wax, foamed polystyrene, natural rubber, styrene-butadiene rubber, nitrile-butadiene rubber, chloroprene rubber, ethylene-propylene rubber, silicone rubber, urethane rubber, fluororubber, phenol resin, and epoxy resin.

The implant fixture of the present invention is applicable as an aritificial tooth root for dentatl purposes and is also applicable as an artificial bone in the fields of orthopedic surgery, plastic surgery, and oral surgery.

These and other objects and many of the attendant advantages of the present invention will readily be appreciated as the same becomes better understood by reference to the following detailed description when considered in connection with the accompanying drawings.

FIG. 1 is an illustration used to explain the shape of an implant fixture of the present invention.

FIG. 2 is an illustration used to explain a manufacturing method of a mold.

FIG. 3 is a perspective view showing a configuration of the mold.

Now, an embodiment of the present invention will be described below in detail with reference to the accompanying drawings.

1. Manufacturing of Implant Fixture

(1) Fabrication of Master Model

SUS (steel use stainless) material is worked into a shape of a pubicly known impant fixture. This is used as a master model. The size of the master model is determined by multiplying the size of a finished implant fixture by a predetermined coeffcient of more than one. This is because the ceramics are shrunk during burning process as described later. The predetermined coefficient differs depending upon the composition of ceramics slurry used. In this embodment, the coefficient is preferably 1.3.

Next, the surface of the master model is subjected to blasting. The surface roughness (arithmetic average roughness Ra and maximum height Rz) of the blasted master model is determined such that a buried portion of the finished implant fixture may have surface roughness, specifically, an arithmetic average roughess Ra of 1 to 5 $\mu$m and maximum height Rz of 5 to 40 $\mu$m. The arithmetic average roughness Ra and maximu height Rz are specified in the "JIS B0601" (2001 edition).

In the Ra range of 1 to 5 $\mu$m, good osseointegration may be obtained. Especially, if Ra is in the range of 1 to 5 $\mu$m and Rz is in the range of 5 to 40 $\mu$m, osseointegration may furthermore be improved.

The surface roughness of the master model that falls within the above-identified range may readily be determined by manufacturing several sorts of implant fixtures having different surface roughness corresponding to varied surface roughness of the master model, and understanding the interrelationship of surface roughness between the master model and finished implant fixture. The arithmetic average roughness Ra and maximum height Rz of the master model may be determined to be as approximately 1.3 times large as those of the finished implant fixture as described earlier.

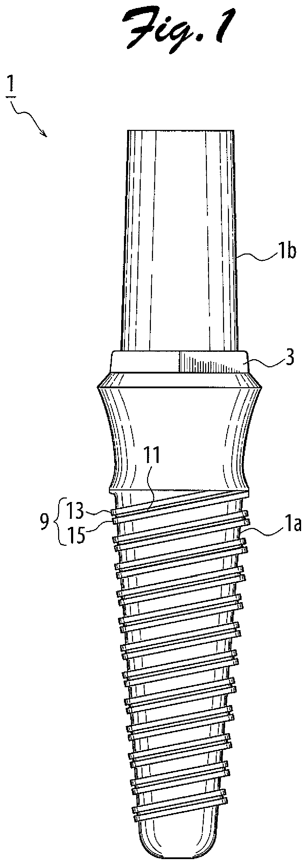

FIG. 1 illustrates the shape of an implant fixture, namely, the master model. The implant fixture 1 has a bar shape as a whole. The implant fixture 1 comprises a buried portion 1a that is to be buried in a living organism and an exposed portion 1b that is exposed out of the living organism and is mounted with a superstructure (not illustrated). The buried portion 1a has a bar shape, more specifically, a cylindrical shape whose diameter becoms smaller toward the tip thereof. A nut portion 3 having a hexagonal section is formed on an outer surface of the buried portion 1a in the vicinity of an upper end of the buried portion 1a. The buried portion 1a is screwed into the living organism by engaging a wrench or spanner with the nut portion 3 and turning the buried portion 1a. A thread pair 9 and a groove 11 are formed in the outer surface of the buried portion 1a except for the nut portion 3. Specifically, the thread pair 9 is spirally formed on the outer surface of the buried portion 1a. The thread pair 9 includes a first thread 13 and a second thread 15 disposed in parallell with a given interval therebetween. The groove 11 is defined as sandwiched between the first and second threads 13,15.

(2) Fabrication of Mold

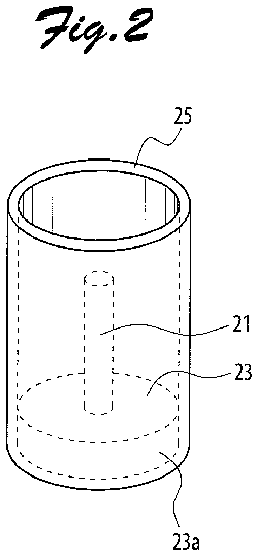

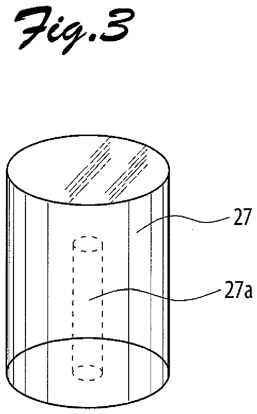

With reference to FIGs. 2 and 3, how to fabricate a mold is described below. As illustrated in FIG. 2, the master model 21 fabricated as described in the above-mentioned (1) is placed on a pedestal 23 having a wider horizontal surface than the master model 21. In FIG. 2, the shape of the master model 21 is simplified. Next, an outer model 25 havig a hollow cylindrical shape with open ends (top and bottom) is mounted around the master model 21 and the pedestal 23 to receive the master model 21 and the pedestal 23 therein. An outer surface 23a of the pedestal 23 is in close contact with an inner surface of the outer model 25 with no gap therebetween.

Next, liquid silicone rubber to be hardened as triggerd by reaction is put into the outer model 25. After 24 hours passes since the liquid rubber has been put into the outer model 25, the mold 27 of the hardened silicone rubber is pulled out of the outer model 25 (see FIG. 2). The mold 27 has a concave portion 27a corresponding to an inverted master model 21 in shape. Since the mold 27 is made of an elastic and strechable material, it can readily be deformed and stretched.

(3) Preparation of Ceramics Slurry

A ceramic slurry is prepared by mixing the following components:

Ceramics powder: 100 parts by mass

Water: 30 parts by mass
Ester resin emulsion (methyl acrylate): 9 parts by mass
Ester based solvnt (butyl carbitol acetate): 3 parts by mass
Ammonia water: To be appropriately added such that the pH of the ceramics slurry may be 9 to 10.

"TZ-3Y-E" (trade name) made by Tosoh Corporation is used as the ceramics powder. "TZ-3Y-E" is mainly composed of zirconia of 93 to 94.9 mass %. It also contains yttria of 4.95 to 5.35 mass % and alumina of 0.15 to 0.35 mass %.

(4) Manufacturing of Implant Fixture

The slurry prepared in the above-mentioned (3) is poured into the concave portion 27a of the mold 27 fabricated in the above-mentioned (2). Then, the mold 27 is heated at 70°C to harden the slurry. The hardened slurry (not-yet-burned ceramics) is pulled out of the mold 27 and is left for 24 hours at ordinary temperature for drying.

Then, the not-yet-burned ceramics are burned at 1300°C to finish an implant fixture. If the burning temperature exceeds 1400°C, the sintered grain size of the zirconia contained in the implant fixture becomes larger or too large in some cases, thereby reducing the durability of the implant fixture. As a result, the implant fixtue is likely to deteriorate due to water, lactic acid, or the like.

2. Evaluation of Finished Implant Fixture

The denseness, monoclinic percentage (percentage of monoclinic crystals), surface roughness, and sintered grain size of the finished implant fixture, which was manufactured by the manufacturing method as describe above, were evaluated. The results are as follows:

Denseness: Relative density of 99 % or more
Monoclinic percentage: 0 volume %
Sintered grain size: 0.15 $\mu$m
Arithmetic average roughness Ra: 1 to 5 $\mu$m
Maximum height Rz: 5 to 40 $\mu$m

The denseness was evaluated by measuring bulk density as specified in JIS R1634 and dividing the value of measured bulk density by theoretical density. The monoclinic percentage was evaluated by X-ray analalysis. The singered grain size was evaluated by planimetric method.

The planimetric method is described below in detail. The sintered suface or mirror polished surface of the ceramics is photographed by a scanning electronic microscope (SEM). A circle having an area A is depicted on the photograh. The number of grains contained in the circle, excluding those grains coinciding on the circumference of the circle, is defined as Na, the number of grains coinciding on the circumference of the circle as Nb, and the magnification of the SEM as M. The average grain size D is calculated as follows and the average grain size thus calculated is considered as the sintered grain size. Number of grains in the circle Nc:

$$Nc = Na + (1/2) \times Nb$$

Number of grains per unit area Ng:

$$Ng = Nc / (A/M^2)$$

Average grain size D:

$$D = \sqrt{(1/Ng)}$$

In this calculation, the sectional shape of a grain is regarded as being square in view of an area of 1/Ng occupied by one grain.

M is set to 8000 or more and the circle is depicted such that the relationship of Nc $\geq$ 100 holds. If such circle cannot be depicted on the photograph, the magnification is decreased and then photogaraphing is performed again. If a circle satisfying the relationship of Nc ≥ 100 cannot be depicted on the phtograph with the magnification of 8000, a pluralrity of photographs that do not overlap each other are taken and a circle is depicted on each photograph. The total Nct of Nc for each circle should sastisfy the relationship of Nct ≥ 100. Then, Ng is calculated as follows:

Number of grains per unit area Ng:

$$Ng = Nct / (At/M^2)$$

where Nct denotes the total of Nc for each circle and At denotes the total of area A for each circle.

The surface roughness is measured by a method conforming to "JIS B0601" (2001 edition).

3. Confirmation Test for Merit (Durability) of Implant Fixture

(1) Preparation of Specimens

(i) Specimen A

Specimen A was prepared by substantially the same method as the method of manufacturing an implant fixutre as mentioned above. Specimen A was a plate in shape having dimensions of 30 mm X 5 mm X 2 mm. The denseness (relative density) of Specimen A was 99 % or more and the sintered grain size thereof was 0.15 μm. The arithmetic average roughness Ra of Specimen A was 1.6 μm and the maximum height Rz thereof was 21 μm.

(ii) Specimen B

Specimen B was prepared by substantially the same method as Specimen A, but the burning temperature was not 1300°C but 1400°C. The denseness (relative density) of Specimen B was 99 % or more and the sintered grain size thereof was 0.28 μm. The arithmetic average roughness Ra of Specimen B was 1.8 μm and the maximum height Rz thereof was 21 μm.

(iii) Specimen C

Specimen C was prepared by substantially the same method as Specimen A, but the burning temperature was not 1300°C but 1550°C. The denseness (relative density) of Specimen C was 99 % or more and the sintered grain size thereof was 0.41 μm. The arithmetic average roughness Ra of Specimen C was 1. 5 μm and the maximum height Rz thereof was 14 μm.

(iv) Specimen R

A precursor was prepared by substantially the same method as Specimen A, but the precursor was a plate in shape having dimensions of 30.1 mm X 5.1 mm X 2.1 mm. One of the surfaces of the precursor was polished with a planar polisher and then subjected to blasting. This surface was a surface of which the monoclinic percentage was measured later. Thus, Specimen R was prepared to have dimensions of 30 mm X 5 mm X 2 mm. Ceramic beads having an average grain size of 280 μm were used as blast media. Blast pressure was 0.5 Kgf/cm$^2$. A pen-type sandblaster was used in blasting.
The denseness (relative density) of Specimen R was 99 % or more and the sintered grain size thereof was 0.15 μm. The arithmetic average roughness Ra of Specimen R was 2.2 μm and the maximum height Rz thereof was 16 μm.

(v) Specimen X

First, Specimen R was prepared. Then, it was subjected to annealing treatment in order to reduce the monoclinic percentage. Thus, Specimen X was prepared. The annealing treatment was performed at a burning temperature of 1000°C for two hours. The denseness (relative density) of Specimen X was 99 % or more and the crystalline grain size thereof was 0.15 μm. The arithmetic average roughness Ra of Specimen X was 2.2 μm and the maximum height Rz

thereof was 22 $\mu$m.

(2) Testing Method

The monoclinic percentage (voume %) was measured in respect of each specimen. Then, each specimen was dipped in a 1% solution of L-lactic acid having a temperature of 35°C. The monoclinic percentage of each specimen was measured one day, ten days, one month, three months, and six months after the dipping was started.

(3) Testing Results

Testing results are shown in Table 1 below.

Table 1

| Specimen | Monoclinic Percentage (volume %) | | | | | |
|---|---|---|---|---|---|---|
| | Before dipping | One day after | 10 days after | One month after | 3 months after | 6 months after |
| A | 0 | 0 | 0 | 0 | 0 | 0 |
| B | 0 | 0 | 0 | 0 | 0 | 0 |
| C | 0 | 0 | 0 | 0 | 2 | 9 |
| R | 3 | 5 | 10 | 20 | 25 | Collapsed |
| X | 0 | 0 | 0 | 2 | 8 | 15 |
| Note: "Collapsed" indicates that the surface of the specimen was collapsed and the monoclinic percentage could not be measured. | | | | | | |

As is clearly known from the table, Specimens A, B, and C each showed much lower monoclinic percentage, compared with Specimen R. Further, the monoclinic percentage of Specimens A, B, and C hardly increased even after the specimens had been dipped in the lactic acid solution for a long time. Especially, Specimens A and B, which were burned at 1400°C or less and had a sintered grain size of 0.3 $\mu$m or less, showed this tendency most.

In contrast with Specimens A and B, Specimen R had a polished surface and showed high initial monoclinic percentage before dipping. The monoclinic percentage of Specimen R rapidly increased while it was dipped in the lactic acid solution, and the surface of Specimen R was collapsed 6 months after the dipping was started.

The monoclinic percentage of Specimens A, B, and C showing low initial monoclinic percentage hardly increased even after they had been dipped in the lactic acid solution. It has been confirmed that Specimens A, B, and C were excellent in durability and that they had appropriate surface roughness.

The implant fixture 1 was actually implanted and used in a living organism. It was excellent in resistance against lactic acid or the like. The implant fixture 1 had high affinity and compatibility with a lliving organism (high bioaffinity and biocompatibility).

4. Confirmation Test for Merit (Osseointegration) of Implant Fixture

(1) Preparation of Specimens

(i) Specimen Aa

Specimen Aa was prepared by substantially the same method as Specimen A. Specimen Aa was substantially the same in shape as the implant fixture as mentioned earlier. The portion to be buried in bone was a screw in shape having a diamenter $\Phi$ of 3.0 mm and a length of 9 mm with a pitch of 1.2 mm and a groove depth of 0.4 mm. The arithmetic average roughness Ra of Specimen Aa was 2.0 $\mu$m and the maximum height Rz thereof was 23 $\mu$m.

(ii) Specimen Ba

Specimen Ba was prepared by substantially the same method as Specimen B. Specimen Ba was substantially the same in shape as Specimen Aa. The arithmetic average roughness Ra of Specimen Ba was 1.8 $\mu$m and the maximum height Rz thereof was 22 $\mu$m.

(iii) Specimen Ca

Specimen Ca was prepared by substantially the same method as Specimen C. Specimen Ca was substantially the same in shape as Specimen Aa. The arithmetic average roughness Ra of Specimen Ca was 1.7 $\mu$m and the maximum height Rz thereof was 18 $\mu$m.

(iv) Specimen Xa

Specimen Xa was prepared by substantially the same method as Specimen X. Specimen Xa was substantially the same in shape as Specimen Aa. The arithmetic average roughness Ra of Specimen Xa was 2.2 $\mu$m and the maximum height Rz thereof was 23 $\mu$m.

(v) Specimen Ya

Specimen Ya was prepared by substantially the same method as Specimen Aa. During the preparation of the specimen, the surface of the master model 21 was not subjected to blasting. The arithmetic average roughness Ra of Specimen Ya was 0.3 $\mu$m and the maximum height Rz thereof was 2 $\mu$m.

(2) Testing Method

Each specimen was implanted in the second mandibular molar of a beagle dog that was one or two years old. Four weeks after, the dog's jawbone having the specimen implanted therein was taken out. Then, the jawbone was fixed and a torque required for removing the implanted specimen from the jawbone was measured. Specifically, the specimen was removed from the jawbone with a driver dedicated for the implant fixture that was connected to a torque meter. The maximum torque detected by the torque meter via the driver was defined as pulling torque strength. Testing was perfomed on each specimen with N = 3.

(3) Testing Results

Measured pulling torque strength of each specimen was shown below. The numeric values shown below are averages when N = 3.

Specimen Aa: 32N·cm (newton centimeter)
Specimen Ba: 29N·cm
Specimen Ca: 28N·cm
Specimen Xa: 32N·cm
Specimen Ya: 16N·cm

The pulling torque strength is a measured value reflecting the achieved osseointegration. As is clearly known from the testing results, the osseointegration differed depending upon the surface roughness. Compared with Specimen Ya having small surface roughness, other specimens having large surface roughness achieved better osseointegration and were stably fixed in the jawbone.
The present invention is not limited to the embodiment described so far but by the scope of the claims. For example, the material of the master model is not limited to SUS, and other metals such as brass may be used.
The implant fixture 1 illustrated in FIG. 1 is a one-piece implant fixture integrally including the buried portion 1a and the exopsed portion 1b. The shape of the implant fixture is not limited to the one illustrated in FIG. 1. Arbitrary shapes may be used. For example, a two-piece implant fixture may be employed, including a separate burined portion and a separate exposed portion. In this case, the buried portion acts as an implant fixtue and the exosed portion acts as an abutment. A female screw is provided in the implant fixture and a male screw is provided in the abutment. The abutment may be fixed onto the implant fixture by screwing the male screw of the abutment into the female screw of the implant fixture.
The manufacturing method of the implant fixture is not liminted to the one described herein. Other methods may be employed. For example, sintered ceramics are ground according to the shape illustrated in FIG. 1 and then subjected to annealing treatment. According to this alternative method, the monoclinic percentage in the sintered ceramics is high immediately after the grinding. The monoclinic percentage may be reduced by annealing treatment. However, the implant fixture manufactured as described earlier has higher resistance against lactic acie or the like than the one manufactured by the alternative method.

**Claims**

1. An implant fixture made from ceramics containing zirconia, wherein:

   the implant fixture comprises at least a buried portion (1a) that is to be buried in a living organism;
   the implant fixture has monoclinic percentage of 1 volume % or less and comprises the buried portion (1a) having an arithmetic average roughness Ra of 1 to 5 $\mu$m; and
   the zirconia content accounts for 86 mass % or more of the total mass of the implant fixture (1).

2. The implant fixture according to claim 1, wherein the implant fixture (1) further contains alumina.

3. The implant fixture according to claim 1 or claim 2, wherein the implant fixture (1) further contains yttria.

4. The implant fixture according to claim 1 or claim 2, wherein the implant fixture (1) has a sintered grain size of 0.45 $\mu$m or less.

5. The implant fixture according to claim 1or claim 2, wherein the implant fixture (1) further contains yttria and has a sintered grain size of 0.45 $\mu$m or less.

**Patentansprüche**

1. Implantat-Befestigungsvorrichtung, die aus einer zirconiumoxidhaltigen Keramik gefertigt ist, worin die Implantat-Befestigungsvorrichtung zumindest einen eingebetteten Abschnitt (1 a) umfasst, der in ein Lebewesen eingebettet werden soll;
   wobei die Implantat-Befestigungsvorrichtung einen monoklinen Prozentsatz von 1 Vol.-% oder weniger aufweist und den eingebetteten Abschnitt (1 a) umfasst, der einen arithmetischen Mittenrauwert Ra von 1 bis 5 $\mu$m aufweist; und
   wobei der Zirconiumoxidanteil 86 Gew.-% oder mehr des Gesamtgewichts der Implantat-Befestigungsvorrichtung (1) ausmacht.

2. Implantat-Befestigungsvorrichtung nach Anspruch 1, worin die Implantat-Befestigungsvorrichtung (1) außerdem Aluminiumoxid enthält.

3. Implantat-Befestigungsvorrichtung nach Anspruch 1 oder Anspruch 2, worin die Implantat-Befestigungsvorrichtung (1) außerdem Yttriumoxid enthält.

4. Implantat-Befestigungsvorrichtung nach Anspruch 1 oder Anspruch 2, worin die Implantat-Befestigungsvorrichtung (1) eine gesinterte Korngröße von 0,45 $\mu$m oder weniger aufweist.

5. Implantat-Befestigungsvorrichtung nach Anspruch 1 oder Anspruch 2, worin die Implantat-Befestigungsvorrichtung (1) außerdem Yttriumoxid enthält und eine gesinterte Korngröße von 0,45 $\mu$m oder weniger aufweist.

**Revendications**

1. Une fixation d'implant réalisée à partir de céramique contenant de la zircone, dans laquelle :

   la fixation d'implant comprend au moins une partie enfoncée (1a) qui doit être enfoncée dans un organisme vivant ;
   la fixation d'implant a un pourcentage monoclinique de 1% en volume ou moins et comprend la partie enfoncée (1a) ayant une rugosité moyenne arithmétique Ra de 1 à 5 $\mu$m ; et
   la teneur en zircone s'élève à 86% en poids ou plus du poids total de la fixation d'implant (1).

2. Fixation d'implant selon la revendication 1, dans laquelle la fixation d'implant (1) comprend en outre de l'alumine.

3. Fixation d'implant selon la revendication 1 ou la revendication 2, dans laquelle la fixation d'implant (1) contient en outre de l'oxyde d'yttrium.

**4.** Fixation d'implant selon la revendication 1 ou la revendication 2, dans laquelle la fixation d'implant (1) a une taille de grain fritté de 0,45 $\mu$m ou moins.

**5.** Fixation d'implant selon la revendication 1 ou la revendication 2, dans laquelle la fixation d'implant (1) contient en outre de l'oxyde d'yttrium et a une taille de grain fritté de 0,45 $\mu$m ou moins.

# Fig.1

# *Fig.2*

*Fig.3*

27

27a

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002362972 A

- EP 2316374 A1

**Non-patent literature cited in the description**

- JIS B0601. 2001

- *JIS B0601,* 2001